# EUROPEAN PATENT APPLICATION

(11) **EP 4 343 325 A1**
(43) Date of publication of application: **27.03.2024**
(21) Application number: 22804700.7
(22) Date of filing: 17.05.2022
(51) Int. Cl.: G01N 33/497

(54) **BIOMARKER FOR INFECTIOUS DISEASE**

(30) Priority: 19.05.2021 JP 2021084723
(71) Applicant: Tohoku University, Sendai-shi, Miyagi 980-8577 (JP); Bio-Xcelerator, Inc., Tokyo 160-0016 (JP)
(72) Inventor: AKAIKE, Takaaki, Sendai-shi, Miyagi 980-8574 (JP); TAKAGI, Satoshi, Tokyo 160-0016 (JP)
(74) Representative: Mewburn Ellis LLP
(86) International application number: PCT/JP2022/020597
(87) International publication number: WO 2022/244789

(57) **Abstract**

The present invention is a biomarker for diagnosing infectious disease wherein the biomarker is a sulfur metabolite, and the sulfur metabolite may be a sulfur metabolite present in exhaled air, and the sulfur metabolite can be detected from exhaled air condensate. The biomarker for infectious disease includes a biomarker for diagnosing bacterial and viral infection, especially new coronavirus infection; a biomarker for diagnosing pneumonias such as infectious pneumonia, especially interstitial pneumonia due to new coronavirus infection, and additionally alveolar pneumonia; and a biomarker for diagnosing exacerbation due to infection, especially for diagnosing the risk of the transition to exacerbation due to new coronavirus infection.

The present invention is further a diagnostic system for detecting a biomarker for diagnosing infection comprising a sulfur metabolite from exhaled air, and is a system for determining infection, especially for diagnosing new coronavirus infection.

## Description

### Technical Field

The present invention relates to a biomarker for infectious disease, and relates to a sulfur metabolite.

### Background Art

The inventor has clarified the physiological function and the metabolic pathway of *in-vivo* reactive sulfur molecular species/active sulfur molecular species (reactive sulfur species, RSS) such as cysteine hydropolysulfide (CysSSH) and glutathione polysulfide (GSSH) (Non Patent Literatures 1 and 2).

Although cysteine hydropolysulfide (CysSSH) is generated in various organisms in large amounts, the biosynthesis and the physiological function thereof are hardly known. It is clear that extensive persulfides are formed in cysteine-containing proteins of *E. coli* and mammalian cells, and it is believed that this is produced in the posttranslational process including chemical reaction related to *in-vivo* sulfur metabolites.

Effective synthesis of CysSSH from the substrate L-cysteine, which is a reaction catalyzed by cysteinyl-tRNA synthetases (CARSs) of procaryotes and mammalians, is present. The targeted disruption of genes encoding mitochondrial CARSs in mouse and human cells shows that CARSs play an important role in endogenous CysSSH production, and suggests that these enzymes function as main cysteine persulfide synthases *in vivo.* CARS also catalyzes the co-translational cysteine persulfidation, and participates in the biosynthesis in mitochondria and the regulation of bioenergy.

Accordingly, the clarification of the CARS-dependent production mechanism of persulfurated metabolites may lead to the understanding of abnormal oxidation-reduction signal transduction under physiological and pathophysiological conditions and suggest therapeutic targets based on oxidative stress and mitochondrial dysfunction.

The chemical properties of polysulfide are not completely understood or clarified due to the reactivity or the complicated redox activity characteristics thereof. The inventor has however developed active sulfur metabolomic analysis using RSS metabolism profiling. This is revealing the *in-vivo* kinetics of RSSs endogenously and ubiquitously generated in both procaryotes and eukaryotes. RSSs, namely active sulfur molecules, maintain energy metabolism in mitochondria (Non Patent Literature 2), and meanwhile exhibit strong antioxidant function (Non Patent Literature 1), antiinflammatory function (Non Patent Literature 3), and immune regulatory function (Non Patent Literature 3).

The pathosis of new coronavirus (SARS-CoV-2), which causes new coronavirus infectious disease (COVID-19), urgently needs to be clarified, and methods for preventing and treating new coronavirus infectious disease urgently needs to be established. SARS-CoV-2 protease inhibitors have been reported as therapeutic drugs for COVID-19 (Non Patent Literatures 4 and 5).

### Citation List

### Non Patent Literature

Non Patent Literature 1
   Ida T, Sawa T, Ihara H, Tsuchiya Y, Watanabe Y, Kumagai Y, Suematsu M, Motohashi H, Fujii S, Matsunaga T, Yamamoto M, Ono K, Devarie-Baez NO, Xian M, Fukuto JM, Akaike T. Reactive cysteine persulfides and S-polythiolation regulate oxidative stress and redox signaling. Proc Natl Acad Sci USA 111:7606-7611 (2014).
Non Patent Literature 2
   Akaike T, Ida T, Wei FY, Nishida M, Kumagai Y, Alam MM, Ihara H, Sawa T, Matsunaga T, Kasamatsu S, Nishimura A, Morita M, Tomizawa K, Nishimura A, Watanabe S, Inaba K, Shima H, Tanuma N, Jung M, Fujii S, Watanabe Y, hmuraya M, Nagy P, Feelisch M, Fuk to JM, Motohashi H. Cysteinyl-tRNA synthetase governs cysteine polysulfidation and mitochondrial bioenergetics. Nat Commun 8:1177 (2017).
Non Patent Literature 3
   Zhang T, Ono K, Tsutsuki H, Ihara H, Islam W, Akaike T, Sawa T. Enhanced cellular polysulfides negatively regulate TLR4 signaling and mitigate lethal endotoxin shock. Cell Chem Biol 26:686-698 (2019).
Non Patent Literature 4
   Zhang L, et al. Crystal structure of SARS-CoV-2 main protease provides a basis for design of improved α ketoamide inhibitors. Science 368:409-412 (2020).
Non Patent Literature 5
   Jin Z, et al. Structure of Mpro from SARS-CoV-2 and discovery of its inhibitors. Nature 582:289-293 (2020).

### Summary of Invention

### Technical Problem

The present inventor has developed a drug that takes the effect of preventing and treating viral infectious disease by inhibiting protease derived from virus, namely an "antiviral drug containing an active sulfur compound as the main medicinal effective ingredient", based on various bioactive actions of active sulfur compounds that are present *in vivo* (Japanese Patent Application No. 2020-167343). An object of the present invention is to achieve the usefulness of a sulfur metabolite based on active sulfur metabolomics.

### Solution to Problem

The present inventor has found that a sulfur metabolite functions as a biomarker related to infectious disease in the examination of the usefulness of the sulfur metabolite in association with the antiviral effect of an activity sulfur compound based on active sulfur metabolomics.

That is, the present invention is characterized by being a biomarker for infection containing the sulfur metabolite. The sulfur metabolite may be a sulfur metabolite present in exhaled air. The sulfur metabolite can be detected from exhaled air condensate. The sulfur metabolite may be especially at least one type of sulfite ions (HSO₃⁻), thiosulfate ions (HS₂O₃⁻), and hydrogen disulfide ions (HS₂⁻); salts thereof; or compounds or molecules containing these; or derivatives thereof. The biomarker for infectious disease includes a biomarker for diagnosing bacterial and viral infection, especially new coronavirus infection; a biomarker for diagnosing pneumonia such as infectious pneumonia, especially interstitial pneumonia due to new coronavirus infection, and additionally alveolar pneumonia; and a biomarker for diagnosing exacerbation due to infection, especially for diagnosing the risk of the transition to exacerbation due to new coronavirus infection.

The present invention is further a diagnostic system for detecting a biomarker for diagnosing infection comprising a sulfur metabolite from exhaled air, and is a system for determining infection, especially for diagnosing new coronavirus infection, including: an exhaled air collector and an exhaled air collecting apparatus for collecting exhaled air of a subject, and an analysis apparatus for the qualitative and quantitative analysis of a sulfur metabolite in the collected exhaled air.

### Advantageous Effect of Invention

According to the present invention, the usefulness of a sulfur metabolite was able to be achieved based on active sulfur metabolomics.

### Brief Description of Drawings

[Figure 1] Figure 1 is a subject list of 12 healthy persons not infected with new coronavirus.
[Figure 2] Figure 2 is a subject list of 12 patients infected with new coronavirus.
[Figure 3] Figure 3 is data on the results of the quantitative analysis of the healthy persons and the patients infected with new coronavirus.
[Figure 4] Figure 4 is the results of measuring sulfite ions (HSO₃⁻) and thiosulfate ions (HS₂O₃⁻) of one patient infected with new coronavirus at the time of the exacerbation.

### Description of Embodiments

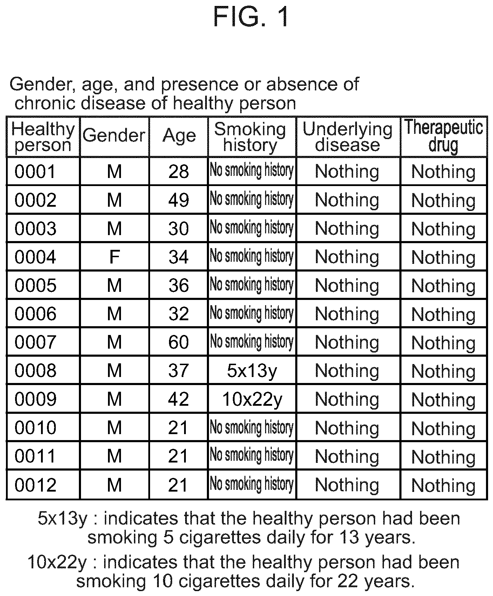

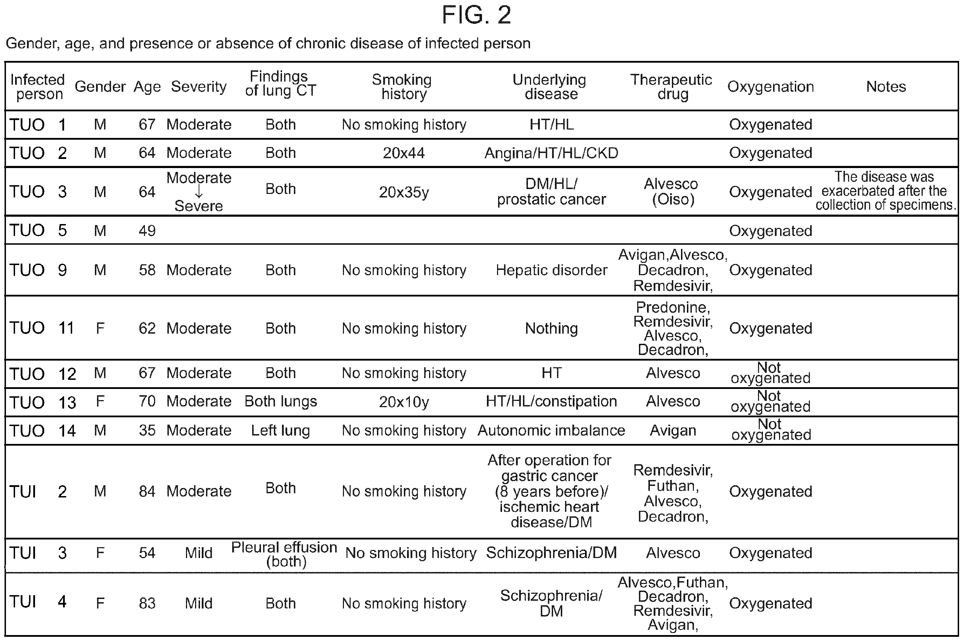

First, 12 healthy persons not infected with new coronavirus (Figure 1) and 12 persons infected with new coronavirus (inpatients in the hospital affiliated with the medical faculty of Tokai University, Figure 2) were analyzed for sulfur metabolites in exhaled air as subjects. It was determined based on the PCR test whether the subjects were infected with new coronavirus or not. Among the twelve persons affected with new coronavirus, two persons were mild, and ten persons were moderate. Among the ten persons, one person was exacerbated later. The classification into mild, moderate, and severe patients was based on the saturated oxygen concentrations, the respiratory symptoms, and the chest CT images. For example, this classification is described in Shingata Korona Wirusu Kansensho (COVID-19), Shinryo no Tebiki Dai 4.1 ban (New Coronavirus Infectious Disease (COVID-19), Medical care manual ver. 4.1).

An exhaled air collector (GL Sciences Inc.) and an exhaled air collecting apparatus (GL Sciences Inc.) were utilized for collecting exhaled air. The exhaled air collecting apparatus was cooled to -20 degrees Celsius beforehand. When a subject breathed for 5 to 10 minutes through the exhaled air collector (mouthpiece type, mask type), exhaled air aerosol was rapidly cooled to collect around 1 ml of exhaled air condensate.

The addition of β-(4-hydroxyphenyl)ethyl iodoacetamide (HPE-IAM), which was an electrophilic alkylating agent, to 25 µl of the exhaled air condensate to 5 mM leads to alkylation reaction at 37 degrees Celsius for 30 minutes. Formic acid was then added to 1% to stabilize sulfur metabolites as HPE-IAM adducts. The electrophilic alkylating agent alkalinizes electron pairs of sulfur, and suppresses the decomposition of the sulfur metabolites.

Furthermore, a stable isotope-labeled HPE-IAM adduct standard was added to each of the multiple sulfur metabolites so that the concentration in the solution was 10 nM. This was prepared into a sample for mass spectrometry. The HPE-IAM adducts of sulfite ions (HSO₃⁻), thiosulfate ions (HS₂O₃⁻), hydrogen disulfide ions (HS₂⁻) were quantitatively analyzed with a mass spectroscope according to Non Patent Literature 1 (Akaike T, et al. Nat Commun 2017). That is, 35 µl of the above-mentioned sample for mass spectrometry/standard was injected into the high-performance liquid chromatographmass spectroscope LCMS(TM)-8060 (SHIMADZU CORPORATION) and quantitatively analyzed under the following MRM (multiple reaction monitoring) conditions (Table 1).

**[Table 1]**

| Compounds | Polarity | Precursor ion (*mlz*) | Production (*m*/*z*) | Collision energy (V) |
|---|---|---|---|---|
| HSO₃-HPE-IAM adduct | - | 258.1 | 121.0 | 20.0 |
| H[³⁴S]O₃-HPE-IAM adduct | - | 260.1 | 121.0 | 20.0 |
| HS₂O₃-HPE-IAM adduct | - | 290.0 | 208.2 | 14.0 |
| H[³⁴S₂]O₃-HPE-IAM adduct | - | 294.0 | 210.2 | 14.0 |
| Bis-SS-HPE-IAM adduct | + | 420.9 | 121.0 | -23.0 |
| Bis-[³⁴S₂]-HPE-IAM adduct | + | 424.9 | 121.0 | -23.0 |

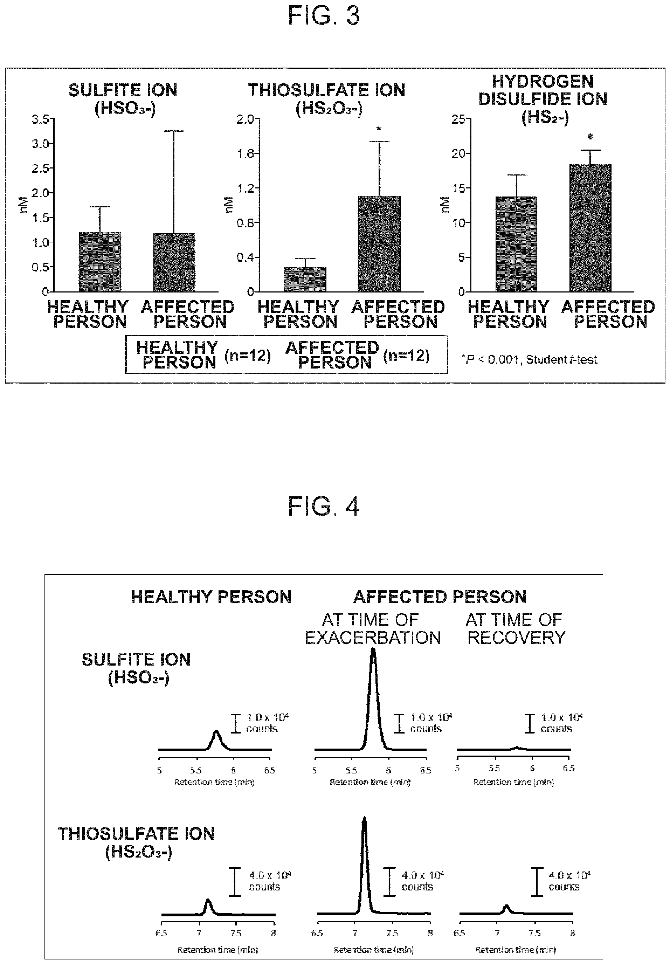

Figure 3 shows data on the results of quantitatively analyzing the 12 healthy persons and the 12 persons infected with new coronavirus for these three types of ions. The concentrations of the various sulfur metabolites contained in the exhaled air condensate were generally at low generation levels as compared with *in vivo.* It is believed that this is influenced by the fact that the *in-vivo* sulfur metabolites are diluted in the exhaled air, and the exhaled air is not necessarily collected efficiently. It was then confirmed that the levels of sulfite ions (HSO₃⁻), thiosulfate ions (HS₂O₃⁻), and hydrogen disulfide ions (HS₂⁻) were significantly high in the moderate patients as compared with the healthy persons.

The symptoms of the patient (patient No. 3) in Figure 2 changed from moderate symptoms to severe symptoms. Figure 4 shows the results of measuring the sulfite ions (HSO₃⁻) and thiosulfate ions (HS₂O₃⁻) at the time of the exacerbation. It was found that the generation levels of thiosulfate ions (HS₂O₃⁻) were significantly high at the time of mild symptoms and also at the time of severe symptoms as compared with the healthy persons. In the moderate patient in which pneumonia was exacerbated later (patient No. 3), a marked increase in sulfite ions (HSO₃⁻) was observed in the specimen collected before the exacerbation. In the moderate patients in which pneumonia was not exacerbated, such an increase was not, however, seen. Accordingly, sulfite ions (HSO₃⁻) are promising as a biomarker for evaluating a risk of the exacerbation of pneumonia due to new coronavirus infectious disease.

Since active sulfur has antioxidant activity, and that is, active sulfur is oxidized by oxidative stress, active oxygen, and the like, it can be said that the exacerbation of pneumonia, namely the aggravation of oxidative stress shifts the profiles of the sulfur metabolites toward the oxidized state.

## Claims

1. A biomarker for diagnosing infectious disease, wherein the biomarker is a sulfur metabolite.

2. The biomarker according to claim 1, wherein the sulfur metabolite is collected from an exhaled air.

3. The biomarker according to claim 2, wherein the sulfur metabolite is produced by metabolism of an active sulfur compound *in vivo.*

4. The biomarker according to claim 3, wherein the sulfur metabolite is at least one type of sulfite ions (HSO₃⁻), thiosulfate ions (HS₂O₃⁻), and hydrogen disulfide ions (HS₂⁻).

5. The biomarker according to claim 4, wherein the biomarker is for diagnosing viral infection.

6. The biomarker according to claim 5, wherein the biomarker is for diagnosing new coronavirus infection.

7. The biomarker according to claim 5 or 6, wherein the biomarker is for diagnosing pneumonia due to the infection.

8. The biomarker according to claim 6, wherein the biomarker is for evaluating a risk of exacerbation of the new coronavirus infection.

9. The biomarker according to claim 8, wherein the sulfur metabolite is sulfite ions (HSO₃⁻).
